# EUROPÄISCHE PATENTANMELDUNG

(11) **EP 2 592 270 A1**
(43) Veröffentlichungstag der Anmeldung: **15.05.2013**
(21) Anmeldenummer: 11188816.0
(22) Anmeldetag: 11.11.2011
(51) Int. Cl.: F04B 43/00, F04B 43/08, F04B 43/12, F04B 45/06, F04B 45/08, A61M 39/10, F16L 33/00

(54) **Pumpenschlauch für eine peristaltische Pumpe**

(71) Anmelder: Connectors Verbindungstechnik AG, 8317 Tagelswangen (CH)
(72) Erfinder: Di Leo, Vito, 8302 Kloten (CH)
(74) Vertreter: Schmauder & Partner AG Patent- & Markenanwälte VSP

(57) **Zusammenfassung**

TBD

## Beschreibung

### Technisches Gebiet

Die Erfindung betrifft einen Pumpenschlauch, insbesondere einen Silikonpumpenschlauch, für eine peristaltische Pumpe.

### Stand der Technik

Peristaltische Pumpen dienen dazu, durch einen Quetschvorgang durch einen Pumpenschlauch eine Vorwärtsbewegung einer Flüssigkeit zu bewirken. Solche peristaltische Pumpen umfassen ein Pumpensegment, in das der Schlauch üblicherweise eingelegt wird. Das Pumpensegment ist dann mit einer Antriebseinrichtung für den peristaltischen Quetschvorgang versehen. Der pumpenschlauch weist üblicherweise Anschlusseinrichtungen auf, die an die Elemente zum Versorgen der Pumpe mit der Flüssigkeit und dem Abnehmer versehen sind. Der Vorteil einer solchen peristaltischen Pumpe ist es, dass wesentliche Teile des Pumpensegmentes, insbesondere das Antriebssegment, nicht mit der peristaltisch zu pumpenden Flüssigkeit in Verbindung kommen. Dies ist besonders vorteilhaft auf dem Gebiet der Biotechnologie, bei der die zu pumpende Flüssigkeit steril sein soll. Aber auch bei kontaminierten Flüssigkeiten auf dem Gebiet der Biotechnologie oder auch in der Technik radioaktiv kontaminierter Flüssigkeiten ist eine solche peristaltische Pumpe vorteilhaft.

Der Pumpenschlauch ist dabei üblicherweise als Wegwerfteil ausgebildet, der normalerweise steril angeliefert wird und nach einer allfälligen Kontamination nicht weiterverwendet werden muss.

Aus der US 5 213 483 A ist eine peristaltische Pumpe mit einem Pumpenkopfgehäuse der genannten Art bekannt. Aus der US 5 388 972 A ist ein Präzisionspumpenkopf bekannt.

In der EP 1 0 48 848 A1 wird ein peristaltischer Pumpenkopf vorgeschlagen, bei dem der Flansch fluiddicht in einer Ausnehmung eingepasst ist. Dies hat sich aber nicht in jedem Fall als vorteilhaft herausgestellt, da dabei bestimmte Belastungen der peristaltischen Pumpe auftreten können, die vermieden werden können.

Andererseits hat sich die Ausführung der EP 1 048 848 A1 als sehr nachteilig herausgestellt. Dort wird nämlich vorgeschlagen, dass ein Endfitting, also ein Anschlussteil auf den Pumpenschlauch aufgespritzt oder an diesen angebracht wird. Damit sind aber - innerhalb des Schlauches - Übergänge nicht zu vermeiden. Diese sollen aber - als Teil der Aufgabe der Erfindung - zumindest vermeidbar sein sollen.

### Darstellung der Erfindung

Eine Aufgabe der vorliegenden Erfindung ist es, einen gegenüber dem Stand der Technik verbesserten Pumpenschlauch vorzuschlagen, der für eine peristaltische Pumpe geeignet ist und durch den Übergänge im Inneren des Schlauches vermeidbar sind. Die Aufgabe der Erfindung wird durch den Pumpenschlauch nach Anspruch 1 gelöst. Dabei haben die Massnahmen der Erfindung zunächst einmal zur Folge, dass kein Aufspritzen oder Anbringen eines Anschlussteils (Fitting) notwendig ist und die oben beschriebenen Probleme gelöst sind. Die Ausgestaltung des Pumpenschlauches aus Silikon, vorzugsweise aus platinvernetztem Silikon, führt zu einer Erhöhung der Qualität. Dadurch, dass die Anschlussteile jeweils kopfartig eine Dichtfläche aufweisen und die Dichtfläche mit einer im Wesentlichen kreisförmigen Dichtwulst umgeben ist, die longitudinal über die Dichtfläche herausragt, ist ein Anbringen der Anschlussteile an die Elemente zum Versorgen der Pumpe mit der Flüssigkeit und dem Abnehmer direkt und besonders vorteilhaft möglich.

Vorteilhafte Ausgestaltungen der erfindungsgemässen Antihaftschicht sind in den abhängigen Ansprüchen 2 bis 5 angegeben.

Vorteilhaft ist es, wenn das Innere des Pumpenschlauches übergangsfrei zwischen den beiden Dichtflächen ausgebildet ist. Selbstverständlich ist dies nicht notwendig. Möglich wäre es auch, bestimmte Übergänge vorzusehen, wenn dies ausnahmsweise gewünscht wird.

Zur Vermeidung von Druckschwankungen etc. kann es vorteilhaft sein, wenn die Flanschteile Mittel zum nicht gasdichten, vorzugsweise auch zum nicht fluiddichten Einlegen des Pumpenschlauches in das peristaltische Pumpensegment aufweisen. Solche Mittel können beispielweise zum schlauchförmigen Mittelteil hin ausgebildete Noppen umfassen, die einen kleinen Schlupf sicherstellen.

Zur Erleichterung des Anschlusses die an die Elemente zum Versorgen der Pumpe mit der Flüssigkeit und dem Abnehmer wird der Pumpenschlauch vorteilhafter Weise mit einem die Dichtwulst zumindest teilweise überragenden Haltering, vorzugsweise aus einem Hartkunststoff versehen. Dieser Haltering aus hartem Material kann dann axial nach vorne bzw. hinten die Dichtwulst und damit die Dichtfläche an die entsprechende Gegenfläche der Elemente zum Versorgen der Pumpe mit der Flüssigkeit und des Abnehmers drücken, ohne dass eine direkte Krafteinwirkung auf den weichen Silikonschlauch ausgeübt werden müsste.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Figuren

Ausführungsbeispiele der Erfindung werden nachfolgend anhand von Zeichnungen näher beschrieben, dabei zeigen:
- Figur 1: eine Seitenansicht eines erfinderischen Pumpenschlauches,
- Figur 2: eine Detailansicht eines der Flanschteile mit dem Anschlussteil gemäss Figur 1, von der Seite;
- Figur 3: eine Detailansicht von Figur 2 mit dem Flanschteil mit den Noppen, von der Seite; und
- Figur 4: eine Sicht auf den Flanschteil des Pumpenschlauches vom Mittelteil aus gesehen, mit den Abstandsnoppen.

### Wege zur Ausführung der Erfindung

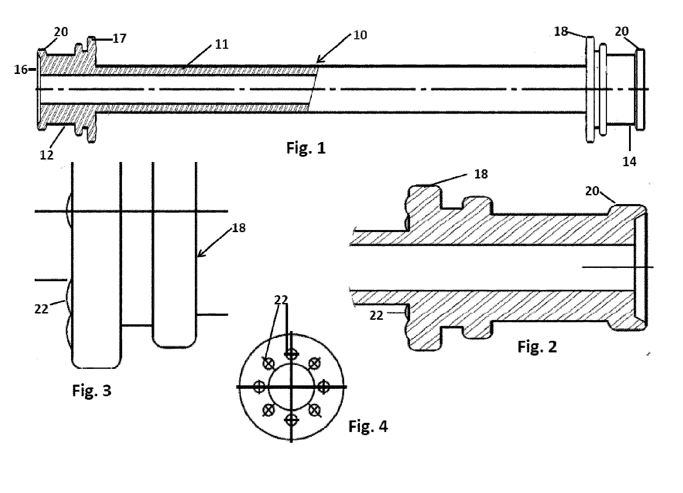

Gemäss einem bevorzugten Ausführungsbeispiel, wie es in Figur 1 dargestellt ist, zeigt einen Pumpenschlauch 10, nämlich einen Silikonpumpenschlauch für eine peristaltische Pumpe. Der Pumpenschlauch 10 besteht grundsätzlich aus fünf Teilbereichen, nämlich

einen schlauchförmigen Mittelteil 11, beidseitig angeordnete Anschlussteile 12 und 14, zum Anschluss des Pumpenschlauches 10 an weitere Elemente, nämlich an die Elemente zum Versorgen der Pumpe mit der Flüssigkeit und dem Abnehmer der gepumpten Flüssigkeit, die nicht dargestellt sind und beidseitig angeordnete Flanschteile 17 und 18 zum Einlegen in einen Kassettenteil der peristaltischen Pumpe.

Alle fünf Teilbereiche des Silikonpumpenschlauch, nämlich der schlauchförmige Mittelteil 11, die beidseitig angeordneten Anschlussteile 12 und 14 und die beidseitig zwischen dem Mittelteil und den Anschlussteilen angeordneten Flanschteilen 17 und 18 sind einstückig aus Silikon, im vorliegenden Ausführungsbeispiel aus platinvernetztem Silikon hergestellt. Dadurch ist der Silikonpumpenschlauch besonders gut sterilisierbar, insbesondere durch Flammensterilisation, ohne dass z.B. aufgespritzte oder anders angebrachte Anschlussteile (Fittings) sich lösen oder sonst wie beschädigt werden könnten. Ausserdem können durch die einstückige Herstellung Übergänge vermieden werden. Durch die einstückige Herstellungsweise ist es zudem möglich, wie im vorliegenden Ausführungsbeispiel die Anschlussteile 12 und 14 jeweils kopfartig jeweils mit einer Dichtfläche 16 zu versehen. Da die Dichtflächen 16 Teil des einstückigen Schlauches sind, kann auf Dichtringe, die sich bei den Schläuchen gemäss dem Stand der Technik als kontaminationsträchtig - vor und nach der Benutzung - verzichtet werden. Im Ausführungsbeispiel sind die Dichtflächen 16 mit einer im Wesentlichen kreisförmigen Dichtwulst 20 umgeben, die longitudinal über die Dichtfläche 16 herausragt.

Im Ausführungsbeispiel ist die Dichtwulst 20 ist gegenüber der Dichtfläche, also nach innen hin, um ca. 70°angefast.

Die Flanschteile 17 und 18 sind im Ausführungsbeispiel ca. 35 mm von der Dichtfläche entfernt, gemessen an der der Dichtfläche angewandten Flächen. Diese, der Dichtfläche angewandten Flächen der Flanschteile 17 und 18 weisen im vorliegenden Ausführungsbeispiel Mittel auf zum nicht gasdichten und auch nicht fluiddichten Einlegen des Pumpenschlauches 10 in das peristaltische Pumpensegment. Damit werden für die durch die Elastizität des Materials, also des Silikons und der damit flexiblen Längen entstehenden Bewegungen, insbesondere bei den peristaltischen Bewegungen, möglicherweise auftretenden Verspannungen vermieden oder vermindert. Im vorliegenden Ausführungsbeispiel sind diese Mittel durch Noppen 22 , nämlich acht im Umfang verteilte Noppen mit eine Erhebung von ca. 0.5 bis 1 mm ausgebildet.

Im vorliegenden Ausführungsbeispiel ist ein die Dichtwulst 20 teilweise überragenden Haltering (nicht dargestellt) aus einem Hartkunststoff zum Befestigen des Pumpenschlauches 10 an die weitere Elemente der peristaltischen Pumpe vorgesehen. Der Haltering ist verschiebbar zwischen dem Flanschteil 17 bzw. 18 und der jeweiligen Dichtwulst 20 angeordnet. Durch diesen Haltering können beim Anschliessen auftretende, das Silikonmaterial möglicherweise schädigende Kräfte vermieden werden, da diese vom Haltering aufgenommen werden.

### Bezugszeichenliste

- 10: Pumpenschlauch
- 11: Mittelteil
- 12: Anschlussteil
- 14: Anschlussteil
- 16: Dichtfläche
- 17: Flanschteil
- 18: Flanschteil
- 20: Dichtwulst
- 21: das Innere des Pumpenschlauches
- 22: Noppen

## Patentansprüche

1. Pumpenschlauch (10), insbesondere Silikonpumpenschlauch, für eine peristaltische Pumpe, zum Einlegen in ein peristaltisches Pumpensegment mit
- einem schlauchförmigen Mittelteil (11),
- beidseitig angeordneten Anschlussteilen (12, 14), zum Anschluss des Pumpenschlauches (10) an weitere Elemente,
- beidseitig angeordneten Flanschteilen (17, 18), insbesondere zum Einlegen in einen Kassettenteil der peristaltischen Pumpe,
**dadurch gekennzeichnet, dass**
- der schlauchförmige Mittelteil (11), die beidseitig angeordneten Anschlussteile (12, 14) und die beidseitig angeordneten Flanschteile (17, 18) einstückig aus Silikon, vorzugsweise aus platinvernetztem Silikon, hergestellt sind,
- die Anschlussteile (12, 14) jeweils kopfartig eine Dichtfläche (16) aufweisen,
- wobei die Dichtfläche (16) mit einer im Wesentlichen kreisförmigen Dichtwulst (20) umgeben ist, die longitudinal über die Dichtfläche (16) herausragt.

2. Pumpenschlauch nach Anspruch 1, **dadurch gekennzeichnet, dass** das Innere (21) des Pumpenschlauches übergangsfrei zwischen den beiden Dichtflächen (16) ausgebildet ist.

3. Pumpenschlauch nach Anspruch 1 oder 2, **dadurch gekennzeichnet, dass** die Flanschteile (17, 18) Mittel zum nicht gasdichten, vorzugsweise auch zum nicht fluiddichten Einlegen des Pumpenschlauches (10) in das peristaltische Pumpensegment aufweisen.

4. Pumpenschlauch nach Anspruch 3, **dadurch gekennzeichnet, dass** die genannten Mittel zum schlauchförmigen Mittelteil (11) hin ausgebildete Noppen (22) umfassen.

5. Pumpenschlauch nach einem der Ansprüche 1 bis 4, **gekennzeichnet durch** einen die Dichtwulst (20) zumindest teilweise überragenden Haltering, vorzugsweise aus einem Hartkunststoff, zum Befestigen des Pumpenschlauches (10) an die weitere Elemente der peristaltischen Pumpe, , dass das Innere (21) des Pumpenschlauches übergangsfrei zwischen den beiden Dichtflächen (16) ausgebildet ist, wobei der Haltering verschiebbar zwischen dem Flanschteil (17, 18) und der Dichtwulst angeordnet ist.
